# EUROPEAN PATENT SPECIFICATION

(11) **EP 2 973 347 B1**
(45) Date of publication and mention of the grant of the patent: **11.12.2019**
(21) Application number: 14767733.0
(22) Date of filing: 05.03.2014
(51) Int. Cl.: G06Q 50/00, A61B 5/00, G06F 3/048

(54) **MULTIPLE PATIENT EEG MONITORING**
MULTIPATIENTEN-EEG-ÜBERWACHUNG
SURVEILLANCE EEG DE PLUSIEURS PATIENTS

(30) Priority: 15.03.2013 US 201313831609
(43) Date of publication of application: 20.01.2016
(73) Proprietor: Persyst Development Corporation, San Diego, CA 92130 (US)
(72) Inventor: NIERENBERG, Nicolas, San Diego, California 92130 (US); WILSON, Scott, B., San Diego, California 92130 (US); SCHEUER, Mark, L., San Diego, California 92130 (US); PIROZHENKO, Andrey, San Diego, California 92130 (US)
(74) Representative: Tomkins & Co
(86) International application number: PCT/US2014/020498
(87) International publication number: WO 2014/149709

(56) References cited:
- US-A1- 2003 055 356
- US-A1- 2006 161 072
- US-A1- 2009 054 743
- US-A1- 2013 044 111
- US-B2- 6 749 566

## Description

### Technical Field

The present invention generally relates to a method and system for displaying EEG data. More specifically, the present invention relates to a method and system for acquiring and monitoring multiple EEG machines.

### Background Art

An electroencephalogram ("EEG") is a diagnostic tool that measures and records the electrical activity of a person's brain in order to evaluate cerebral functions. Multiple electrodes are attached to a person's head and connected to a machine by wires. The machine amplifies the signals and records the electrical activity of a person's brain. The electrical activity is produced by the summation of neural activity across a plurality of neurons. These neurons generate small electric voltage fields. The aggregate of these electric voltage fields create an electrical reading which electrodes on the person's head are able to detect and record. An EEG is a superposition of multiple simpler signals. In a normal adult, the amplitude of an EEG signal typically ranges from 1 micro-Volt to 100 micro-Volts, and the EEG signal is approximately 10 to 20 milli-Volts when measured with subdural electrodes. The monitoring of the amplitude and temporal dynamics of the electrical signals provides information about the underlying neural activity and medical conditions of the person.

An EEG is performed to: diagnose epilepsy; verify problems with loss of consciousness or dementia; verify brain activity for a person in a coma; study sleep disorders, monitor brain activity during surgery, and additional physical problems.

Multiple electrodes (typically 17-21, however there are standard positions for at least 70) are attached to a person's head during an EEG. The electrodes are referenced by the position of the electrode in relation to a lobe or area of a person's brain. The references are as follows: F = frontal; Fp = frontopolar; T = temporal; C = central; P = parietal; O = occipital; and A = auricular (ear electrode). Numerals are used to further narrow the position and "z" points relate to electrode sites in the midline of a person's head. An electrocardiogram ("EKG") may also appear on an EEG display.

The EEG records brain waves from different amplifiers using various combinations of electrodes called montages. Montages are generally created to provide a clear picture of the spatial distribution of the EEG across the cortex. A montage is an electrical map obtained from a spatial array of recording electrodes and preferably refers to a particular combination of electrodes examined at a particular point in time.

In bipolar montages, consecutive pairs of electrodes are linked by connecting the electrode input 2 of one channel to input 1 of the subsequent channel, so that adjacent channels have one electrode in common. The bipolar chains of electrodes may be connected going from front to back (longitudinal) or from left to right (transverse). In a bipolar montage signals between two active electrode sites are compared resulting in the difference in activity recorded. Another type of montage is the referential montage or monopolar montage. In a referential montage, various electrodes are connected to input 1 of each amplifier and a reference electrode is connected to input 2 of each amplifier. In a reference montage, signals are collected at an active electrode site and compared to a common reference electrode.

Reference montages are good for determining the true amplitude and morphology of a waveform. For temporal electrodes, CZ is usually a good scalp reference.

Being able to locate the origin of electrical activity ("localization") is critical to being able to analyze the EEG. Localization of normal or abnormal brain waves in bipolar montages is usually accomplished by identifying "phase reversal," a deflection of the two channels within a chain pointing to opposite directions. In a referential montage, all channels may show deflections in the same direction. If the electrical activity at the active electrodes is positive when compared to the activity at the reference electrode, the deflection will be downward. Electrodes where the electrical activity is the same as at the reference electrode will not show any deflection. In general, the electrode with the largest upward deflection represents the maximum negative activity in a referential montage.

Some patterns indicate a tendency toward seizures in a person. A physician may refer to these waves as "epileptiform abnormalities" or "epilepsy waves." These include spikes, sharp waves, and spike-and-wave discharges. Spikes and sharp waves in a specific area of the brain, such as the left temporal lobe, indicate that partial seizures might possibly come from that area. Primary generalized epilepsy, on the other hand, is suggested by spike-and-wave discharges that are widely spread over both hemispheres of the brain, especially if they begin in both hemispheres at the same time.

There are several types of brain waves: alpha waves, beta waves, delta wave, theta waves and gamma waves. Alpha waves have a frequency of 8 to 12 Hertz ("Hz"). Alpha waves are normally found when a person is relaxed or in a waking state when a person's eyes are closed but the person is mentally alert. Alpha waves cease when a person's eyes are open or the person is concentrating. Beta waves have a frequency of 13Hz to 30Hz. Beta waves are normally found when a person is alert, thinking, agitated, or has taken high doses of certain medicines. Delta waves have a frequency of less than 3Hz. Delta waves are normally found only when a person is asleep (non-REM or dreamless sleep) or the person is a young child. Theta waves have a frequency of 4Hz to 7Hz. Theta waves are normally found only when the person is asleep (dream or REM sleep) or the person is a young child. Gamma waves have a frequency of 30Hz to 100Hz. Gamma waves are normally found during higher mental activity and motor functions.

The following definitions are used herein.

"Amplitude" refers to the vertical distance measured from the trough to the maximal peak (negative or positive). It expresses information about the size of the neuron population and its activation synchrony during the component generation.

The term "analogue to digital conversion" refers to when an analogue signal is converted into a digital signal which can then be stored in a computer for further processing. Analogue signals are "real world" signals (e.g., physiological signals such as electroencephalogram, electrocardiogram or electrooculogram). In order for them to be stored and manipulated by a computer, these signals must be converted into a discrete digital form the computer can understand.

"Artifacts" are electrical signals detected along the scalp by an EEG, but that originate from non-cerebral origin. There are patient related artifacts (e.g., movement, sweating, ECG, eye movements) and technical artifacts (50/60 Hz artifact, cable movements, electrode paste-related).

The term "differential amplifier" refers to the key to electrophysiological equipment. It magnifies the difference between two inputs (one amplifier per pair of electrodes).

"Duration" is the time interval from the beginning of the voltage change to its return to the baseline. It is also a measurement of the synchronous activation of neurons involved in the component generation.

"Electrode" refers to a conductor used to establish electrical contact with a nonmetallic part of a circuit. EEG electrodes are small metal discs usually made of stainless steel, tin, gold or silver covered with a silver chloride coating. They are placed on the scalp in special positions.

"Electrode gel" acts as a malleable extension of the electrode, so that the movement of the electrodes leads is less likely to produce artifacts. The gel maximizes skin contact and allows for a low-resistance recording through the skin.

The term "electrode positioning" (10/20 system) refers to the standardized placement of scalp electrodes for a classical EEG recording. The essence of this system is the distance in percentages of the 10/20 range between Nasion-Inion and fixed points. These points are marked as the Frontal pole (Fp), Central (C), Parietal (P), occipital (O), and Temporal (T). The midline electrodes are marked with a subscript z, which stands for zero. The odd numbers are used as subscript for points over the left hemisphere, and even numbers over the right

"Electroencephalogram" or "EEG" refers to the tracing of brain waves, by recording the electrical activity of the brain from the scalp, made by an electroencephalograph.

"Electroencephalograph" refers to an apparatus for detecting and recording brain waves (also called *encephalograph).*

"Epileptiform" refers to resembling that of epilepsy.

"Filtering" refers to a process that removes unwanted frequencies from a signal.

"Filters" are devices that alter the frequency composition of the signal.

"Montage" means the placement of the electrodes. The EEG can be monitored with either a bipolar montage or a referential one. Bipolar means that there are two electrodes per one channel, so there is a reference electrode for each channel. The referential montage means that there is a common reference electrode for all the channels.

"Morphology" refers to the shape of the waveform. The shape of a wave or an EEG pattern is determined by the frequencies that combine to make up the waveform and by their phase and voltage relationships. Wave patterns can be described as being: "Monomorphic". Distinct EEG activity appearing to be composed of one dominant activity. "Polymorphic", distinct EEG activity composed of multiple frequencies that combine to form a complex waveform. "Sinusoidal". Waves resembling sine waves. Monomorphic activity usually is sinusoidal. "Transient". An isolated wave or pattern that is distinctly different from background activity.

"Spike" refers to a transient with a pointed peak and a duration from 20 to under 70 msec.

The term "sharp wave" refers to a transient with a pointed peak and duration of 70-200 msec.

The term "neural network algorithms" refers to algorithms that identify sharp transients that have a high probability of being epileptiform abnormalities.

"Noise" refers to any unwanted signal that modifies the desired signal. It can have multiple sources.

"Periodicity" refers to the distribution of patterns or elements in time (e.g., the appearance of a particular EEG activity at more or less regular intervals). The activity may be generalized, focal or lateralized.

An EEG epoch is an amplitude of an EEG signal as a function of time and frequency.

There is a need to be able to monitor multiple EEG machines from a single site since a facility such as a hospital, may only have one technician skilled in reading EEG recordings. United States Patent Application Publication No. Us 2013/0044111 relates to a user configurable central monitoring station. United States Patent Application Publication No. US 2006/016072 relates to a system for displaying.

### Summary Of The Invention

The present invention provides for monitoring and analysis of EEG recordings for multiple EEG machines at a central location, as defined in the appended claims. Although, multiple EEG machines are monitored, the present invention allows for each EEG recording to be monitored independently and without affecting the other EEG recordings.

One aspect of the present invention is a method for monitoring multiple EEG machines and acquiring EEG recordings for each of the EEG machines. The method includes monitoring a plurality of EEG machines from a central station. Each of the plurality of EEG machines comprises a plurality of electrodes, an amplifier and processor. The central station is in communication with each of the plurality of EEG machine over a network. The method also includes acquiring EEG signals from each active EEG machine of the plurality of EEG machines. The method also includes processing the EEG signals to generate processed EEG recordings for each of the active EEG machines of the plurality of EEG machines. The method also includes displaying each of the processed EEG recordings for each of the active EEG machines of the plurality of EEG machines in a separate cell on a display page at a client device as defined in claim 11.

Another aspect of the present invention is a system for monitoring multiple EEG machines and acquiring EEG recording from each EEG machine. The system includes multiple EEG machines, a network, and a client site. Each of EEG machines comprises a plurality of electrodes for generating a plurality of EEG signals. The client site comprises a processing engine, a database and a display monitor. The processing engine is configured to process each of the plurality of EEG signals from each of the EEG machines to generate a plurality of processed EEG recordings. The processing engine is configured to display each of the EEG recordings in a separate cell in a display page for display on the display monitor as defined in claim 1.

### Brief Description Of The Drawings

FIG. 1 is a block diagram of a system for monitoring multiple EEG machines and acquiring EEG recordings for each of the EEG machines.
FIG. 2 is an illustration of a multiple cell display page for monitoring multiple EEG machines.
FIG. 2A an enlarge view of dashed line box 2A of FIG. 2.
FIG. 3 is an illustration of a single cell display page for monitoring multiple EEG machines.
FIG. 3A an enlarge view of dashed line box 3A of FIG.3.
FIG. 3B an enlarge view of dashed line box 3B of FIG.3.
FIG. 4 is a block diagram of a system for monitoring multiple EEG machines and acquiring EEG recordings for each of the EEG machines.
FIG. 5 is a flow chart for a method for monitoring multiple EEG machines and acquiring EEG recordings for each of the EEG machines.

### Best Mode(s) For Carrying Out The Invention

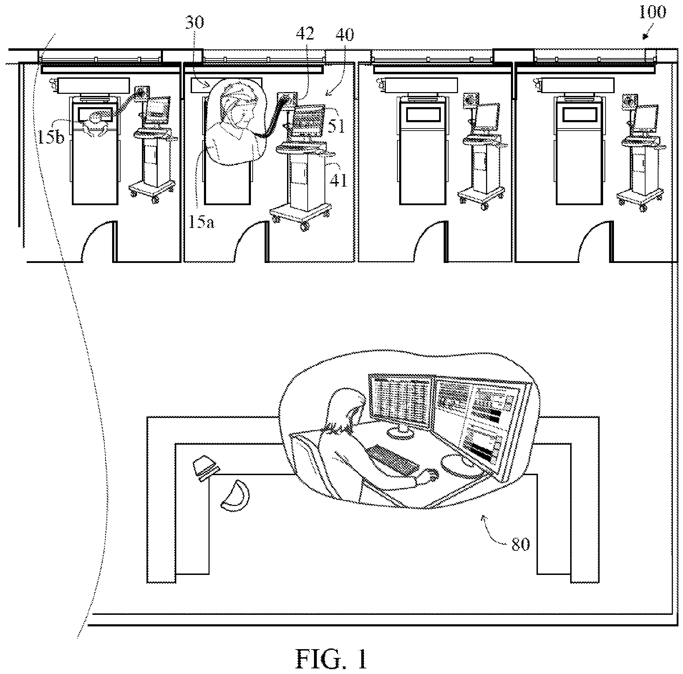

As shown in FIG. 1, a system for monitoring multiple EEG machines and acquiring EEG recordings for each of the EEG machines is generally designated 100. The system 100 comprises a plurality of EEG machines 40, a network and a central station 80. Preferably, each of the EEG machines 40 is located within a single facility along with the central station 80. Alternatively, each of the EEG machines 40 is located at various facilities and connected to the central station over the network.

The system 100 allows for monitoring multiple EEG machines 40 and acquiring EEG recordings for each active EEG machine at the central station 80.

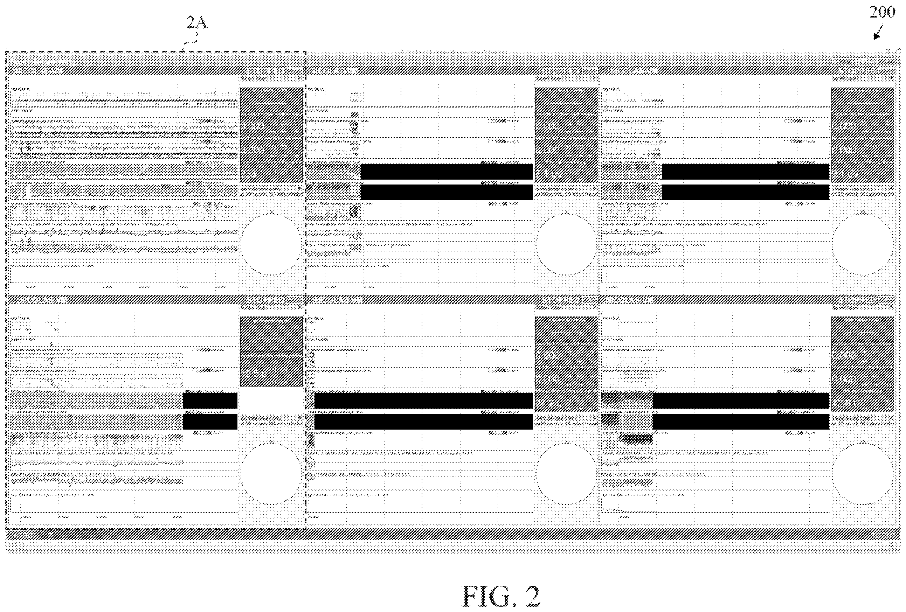

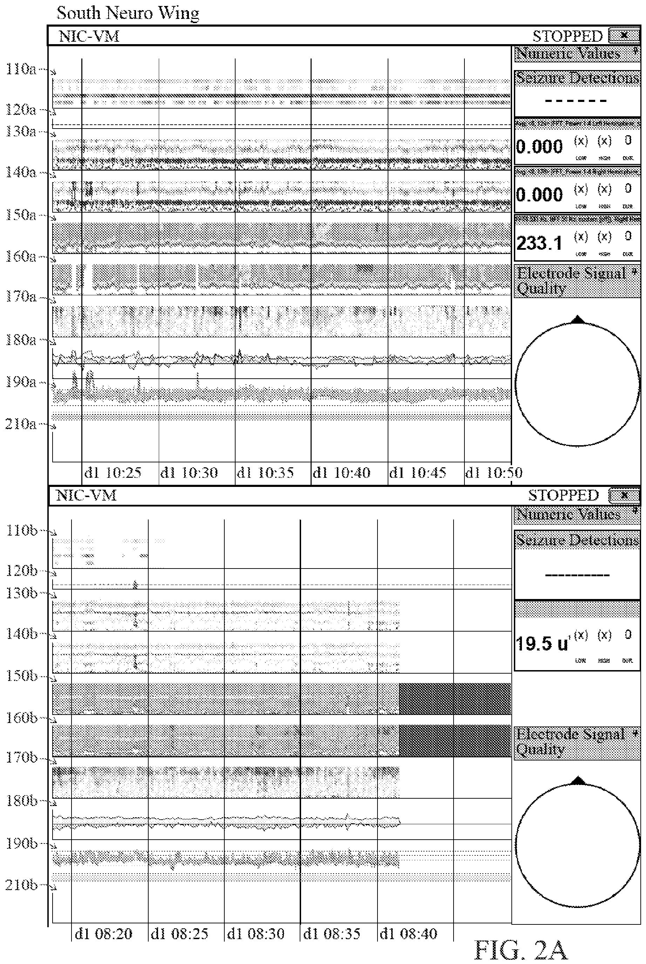

Each of the EEG machines 40 preferably comprises a plurality of electrodes 30, an amplifier 42, a processor 41 and a display 51. FIG. 2 illustrates a system 20 for a user interface for automated artifact filtering for an EEG. A patient 15 wears an electrode cap 31, consisting of a plurality of electrodes 35a-35c, attached to the patient's head with wires 38 from the electrodes 35 connected to an EEG machine component 40 which consists of an amplifier 42 for amplifying the signal to a computer 41 with a processor, which is used to analyze the signals from the electrodes 35 and create an EEG recording 51, which can be viewed on a display 50. A more thorough description of an electrode utilized with the present invention is detailed in Wilson et al. , U. S . Patent Number 8112141 for a Method And Device For Quick Press On EEG Electrode. The EEG is optimized for automated artifact filtering. The EEG recordings are then processed using neural network algorithms to generate a processed EEG recording which is analyzed for display.

A client device at the central station 80 preferably has an EEG processing engine to display each of the EEG recordings for each of the EEG machines 40 in a separate cell in a display page for display on the display monitor at the central station 80. The processing engine is configured to preferably display a plurality of trends for each of the EEG recordings in each of the separate cells in the display page. The processing engine is configured to preferably assign a separate cell to a newly active EEG machine. The processing engine is configured to preferably allow for switching between multiple modes. The processing engine is configured to preferably update each of the separate cells in real-time. The processing engine is configured to preferably permit one-click to open an active EEG recording and occupy the entire display page with the single separate cell. The processing engine is configured to preferably assign a user interface. The processing engine is configured to preferably change a parameter of a plurality of parameters of one cell without affecting the plurality of parameters of any of the other cells on the display page. The processing engine is configured to preferably move through different open EEG recordings on the display page, move to a partial EEG recording. The processing engine is configured to preferably allow for review of an entire EEG record on the display page.

An additional description of analyzing EEG recordings is set forth in Wilson et al, U.S. Patent Application Number 13/620855, filed on September 15, 2012, for a Method And System For Analyzing An EEG Recording.

A patient has a plurality of electrodes attached to the patient's head with wires from the electrodes connected to an amplifier for amplifying the signal to a processor, which is used to analyze the signals from the electrodes and create an EEG recording. The brain produces different signals at different points on a patient's head. Multiple electrodes are positioned on a patient's head. The CZ site is in the center. The number of electrodes determines the number of channels for an EEG. A greater number of channels produce a more detailed representation of a patient's brain activity. Preferably, each amplifier 42 of an EEG machine component 40 corresponds to two electrodes 35 attached to a head of the patient 15. The output from an EEG machine component 40 is the difference in electrical activity detected by the two electrodes. The placement of each electrode is critical for an EEG report since the closer the electrode pairs are to each other, the less difference in the brainwaves that are recorded by the EEG machine component 40. A more thorough description of an electrode utilized with the present invention is detailed in Wilson et al, U.S. Patent Number 8112141 for a Method And Device For Quick Press On EEG Electrode.

FIG. 2 is an illustration of a display page 200. As shown in FIG.2, this display page 20 has six separate cells that each represent a separate EEG machine 40 connected to the client device over a network. Each separate cell can display different trends for each EEG recording from each EEG machine. As shown in FIG. 2A, a separate cell of the display page 200 displays multiple trends for a EEG recording.

As shown in FIG. 2A, the multiple trends can be different for each separate cell. An artifact intensity trend 1 10a is shown as a series of horizontal lines. The plurality of horizontal lines shown comprises a horizontal line for a muscle artifact, a horizontal line for a chewing artifact, a horizontal line for a vertical eye movement artifact, and a horizontal line for a lateral eye movement artifact. Those skilled in the pertinent art will recognize that more or less horizontal lines may be used without departing from the scope of the present invention.

Also shown in FIG.2A for the upper separate cell are a seizure probability trend 120a, a rhythmicity spectrogram, left hemisphere trend 130a, a rhythmicity spectrogram, right hemisphere trend 140a, a FFT spectrogram left hemisphere trend 150a, a FFT spectrogram right hemisphere trend 160a, an asymmetry relative spectrogram trend 170a, a asymmetry absolute index trend 180a, an aEEG trend 190a, and a suppression ration, left hemisphere and right hemisphere trend 210a.

Also shown in FIG.2A are trends for a different separate cell, which include a seizure probability trend 120b, a rhythmicity spectrogram, left hemisphere trend 130b, a rhythmicity spectrogram, right hemisphere trend 140b, a FFT spectrogram left hemisphere trend 150b, a FFT spectrogram right hemisphere trend 160b, an asymmetry relative spectrogram trend 170b, a asymmetry absolute index trend 180b, an aEEG trend 190b, and a suppression ration, left hemisphere and right hemisphere trend 210b.

The display page 200 illustrates that the artifact intensity trend 110a for the upper separate cell has a much great amount of artifacts than the artifact intensity trend 110b of the lower separate cell of the display page 200.

Rhythmicity spectrograms allow one to see the evolution of seizures in a single image. The rhythmicity spectrogram measures the amount of rhythmicity which is present at each frequency in an EEG record.

The seizure probability trend shows a calculated probability of seizure activity over time. The seizure probability trend shows the duration of detected seizures, and also suggests areas of the record that may fall below the seizure detection cutoff, but are still of interest for review. The seizure probability trend when displayed along with other trends, provides a comprehensive view of quantitative changes in an EEG.

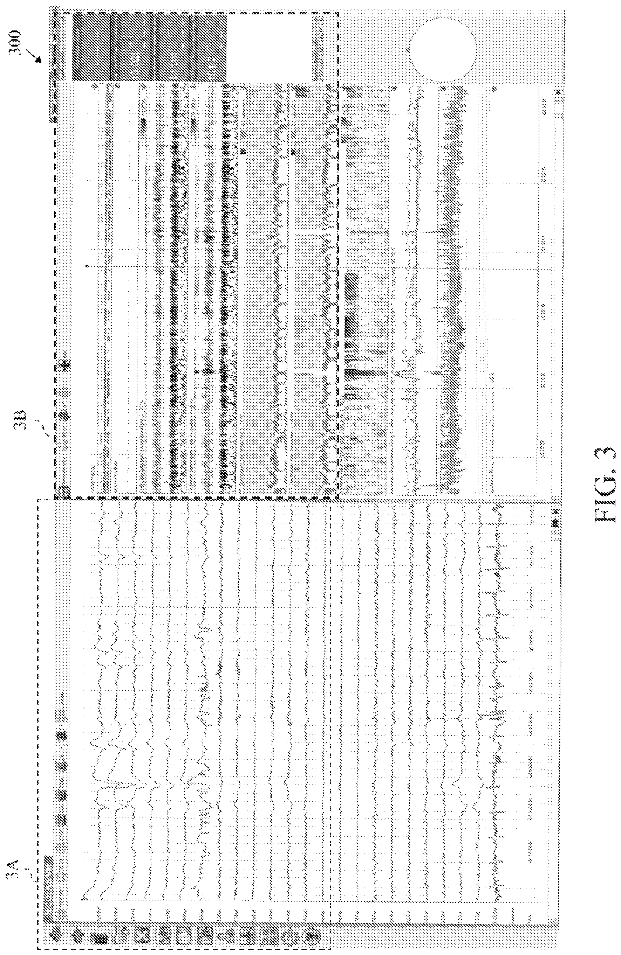

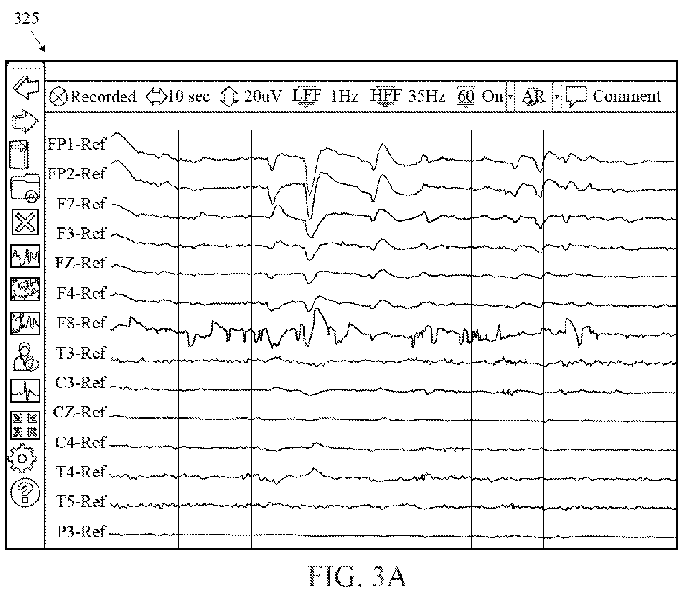

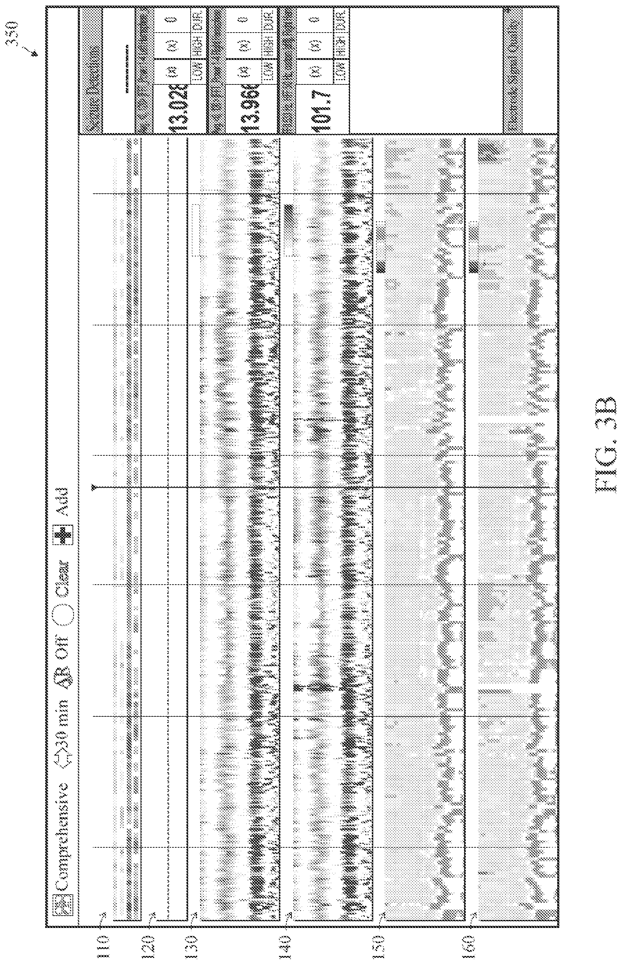

A full display page view of a single cell is shown in FIG. 3. The processing engine allows for a one-click mechanism to move from multiple separate cells on a display page to a full screen view of a single separate cell. Thus, as shown in FIG. 3A and 3B, a real-time EEG recording and trends for a single separate cell are displayed on a single display page.

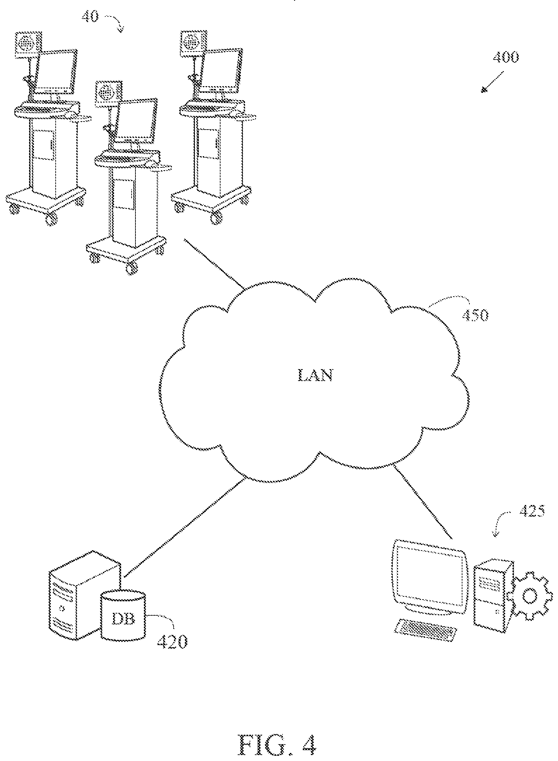

A system 400 for monitoring multiple EEG machines and acquiring EEG recording from each EEG machine is shown in FIG. 4. The system 100 comprises a plurality of EEG machines 40, a network 450 (preferably a local area network), a client site 425 and a database 420 for storing EEG recordings. Preferably, each of the EEG machines 40 is located within a single facility along with the central station 80. Alternatively, each of the EEG machines 40 is located at various facilities and connected to the central station over the network.

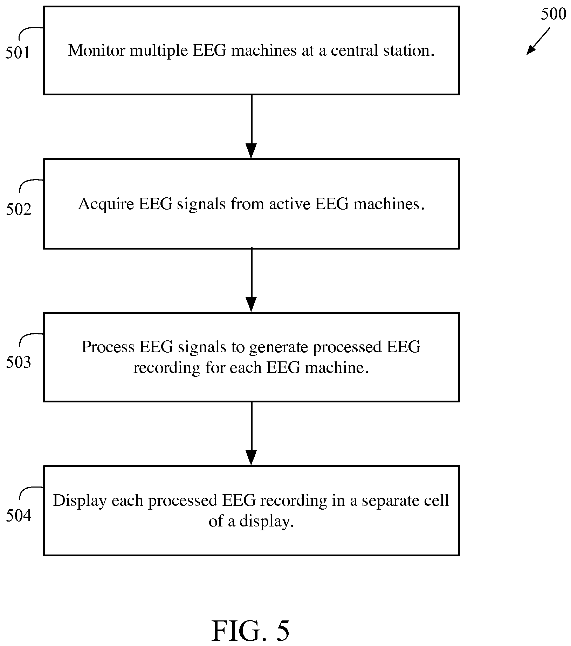

A method 500 for monitoring multiple EEG machines and acquiring EEG recordings for each of the EEG machines is shown in FIG. 5. At block 501, multiple EEG machines are monitored from a central station. Each of the plurality of EEG machines comprises a plurality of electrodes, an amplifier and processor. The central station is in communication with each of the plurality of EEG machine over a network. At block 502, EEG signals are acquired from each active EEG machine of the plurality of EEG machines. At block 503, the EEG signals are processed to generate processed EEG recordings for each of the active EEG machines of the plurality of EEG machines. At block 504, each of the processed EEG recordings for each of the active EEG machines of the plurality of EEG machines is displayed in a separate cell on a display page at a display monitor at a client device.

The EEG is optimized for automated artifact filtering. The EEG recordings are then processed using neural network algorithms to generate a processed EEG recording, which is analyzed for display. During acquisition of the EEG recording, a processing engine performs continuous analysis of the EEG waveforms and determines the presence of most types of electrode artifact on a channel-by-channel basis. Much like a human reader, the processing engine detects artifact by analyzng multiple features of the EEG traces. The preferred artifact detection is independent of impedance checking. During acquisition the processing monitors the incoming channels looking for electrode artifacts. When artifacts are detected they are automatically removed from the seizure detection process and optionally removed from the trending display. This results in much a much higher level of seizure detection accuracy and easier to read trends than in previous generation products.

Algorithms for removing artifact from EEG typically use Blind Source Separation (BSS) algorithms like CCA (canonical correlation analysis) and ICA (Independent Component Analysis) to transform the signals from a set of channels into a set of component waves or "sources."

In one example an algorithm called BSS-CCA is used to remove the effects of muscle activity from the EEG. Using the algorithm on the recorded montage will frequently not produce optimal results. In this case it is generally optimal to use a montage where the reference electrode is one of the vertex electrodes such as CZ in the international 10-20 standard. In this algorithm the recorded montage would first be transformed into a CZ reference montage prior to artifact removal. In the event that the signal at CZ indicates that it is not the best choice then the algorithm would go down a list of possible reference electrodes in order to find one that is suitable.

It is possible to perform BSS-CCA directly on the user-selected montage. However this has two issues. First this requires doing an expensive artifact removal process on each montage selected for viewing by the user. Second the artifact removal will vary from one montage to another, and will only be optimal when a user selects a referential montage using the optimal reference. Since a montage that is required for reviewing an EEG is frequently not the same as the one that is optimal for removing artifact this is not a good solution.

## Claims

1. A system (100, 400) for monitoring multiple EEG machines and acquiring EEG recording from each EEG machine, the system comprising:
a plurality of EEG machines (40), each of the plurality of EEG machine comprising a plurality of electrodes (30) for generating a plurality of EEG signals;
a network (450);
a client site comprising a processing engine, a database (420) and a display monitor, the processing engine configured to process each of the plurality of EEG signals from each of the EEG machines to generate a plurality of processed EEG recordings, wherein the processing engine is configured to display each of the EEG recordings in a separate cell in a display page (200) for display on the display monitor;
**characterised in that** each of the plurality of EEG machines is in a separate facility and the processing engine is configured to monitor each channel for electrode artifacts during acquisition of the EEG signals and remove the electrode artifacts to provide a higher level of seizure detection accuracy than without removal of the electrode artifacts;
wherein the processing engine is configured to detect artifacts in the EEG signals by analyzing a plurality of features;
wherein an artifact intensity (110a) is illustrated in each of the plurality of separate cells, the artifact intensity shown as a series of horizontal lines comprising a horizontal line for a muscle artifact, a horizontal line for a chewing artifact, a horizontal line for a vertical eye movement artifact, and a horizontal line for a lateral eye movement artifact;
wherein the processing engine is configured to display a plurality of trends for each of the EEG recordings in each of the separate cells in the display page, wherein the plurality of trends comprises a seizure probability trend (120a) showing a calculated probability of seizure activity over a predetermined time, a rhythmicity spectrogram measuring the amount of rhythmicity which is present at each frequency in an EEG record, a left hemisphere trend (130a), a right hemisphere trend (140a), a fast Fourier transformation, FFT, spectrogram left hemisphere trend (150a), a FFT spectrogram right hemisphere trend (160a), an asymmetry relative spectrogram trend, and an ambulatory EEG, aEEG trend (190a).

2. The system according to claim 1 wherein the processing engine is configured to assign a separate cell to a newly active EEG machine.

3. The system according to claim 1 wherein the processing engine is configured to allow for switching between multiple modes.

4. The system according to claim 1 wherein the processing engine is configured to update each of the separate cells in real-time.

5. The system according to claim 1 wherein the processing engine is configured to permit one-click to open an active EEG recording and occupy the entire display page with the single separate cell.

6. The system according to claim 1 wherein the processing engine is configured to assign a user interface.

7. The system according to claim 1 wherein the processing engine is configured to change a parameter of a plurality of parameters of one cell without affecting the plurality of parameters of any of the other cells on the display page.

8. The system according to claim 1 wherein the processing engine is configured to move through different open EEG recordings on the display page, move to a partial EEG recording, and permit one-click to open an active EEG recording and occupy the entire display page with the single separate cell.

9. The system according to claim 1 wherein the processing engine is configured to allow for review of an entire EEG record on the display page.

10. The system according to claim 1 wherein each of the EEG machines of the plurality of EEG machines further comprises a processor connected to the plurality of electrodes to generate an EEG recording from the plurality of EEG signals and a display connected to the processor for displaying an EEG recording in proximity to the patient.

11. A method (500) for monitoring multiple EEG machines and acquiring EEG recordings for each of the EEG machines, the method comprising:
Monitoring (501) a plurality of EEG machines from a central station, each of the plurality of EEG machines comprising a plurality of electrodes, an amplifier and processor, the central station in communication with each of the plurality of EEG machine over a network;
acquiring (502) EEG signals from each active EEG machine of the plurality of EEG machines;
transmitting the EEG signals over a network to a processing engine at a client device;
processing (503) the EEG signals at the processing engine to generate processed EEG recordings for each of the active EEG machines of the plurality of EEG machines and a plurality of trends for each of the processed EEG recordings;
displaying (504) each of the processed EEG recordings for each of the active EEG machines of the plurality of EEG machines and at least one trend of the plurality of trends in a separate cell on a display page at a client device;
**characterised in that** each of the plurality of EEG machines is in a separate facility and **characterised by** monitoring, by the processing engine, each channel for electrode artifacts during acquisition of the EEG signals and removing the electrode artifacts to provide a higher level of seizure detection accuracy than without removal of the electrode artifacts;
detecting, by the processing engine, artifacts in the EEG signals by analyzing a plurality of features;
illustrating an artifact intensity in each of the plurality of separate cells, the artifact intensity shown as a series of horizontal lines comprising a horizontal line for a muscle artifact, a horizontal line for a chewing artifact, a horizontal line for a vertical eye movement artifact, and a horizontal line for a lateral eye movement artifact;
wherein the plurality of trends comprises a seizure probability trend showing a calculated probability of seizure activity over a predetermined time, a rhythmicity spectrogram measuring the amount of rhythmicity which is present at each frequency in an EEG record, a left hemisphere trend, a right hemisphere trend, a fast Fourier transformation, FFT, spectrogram left hemisphere trend, a FFT spectrogram right hemisphere trend, an asymmetry relative spectrogram trend, and an ambulatory EEG, aEEG, trend.

12. The method according to claim 11, further comprising assigning a separate cell of the plurality of separate cells to a newly active EEG machine.

13. The method according to claim 11 further comprising switching between multiple modes in each separate cell of the plurality of separate cells.

14. The method according to claim 11 further comprising updating each separate cell of the plurality of separate cells in real-time.

15. The method according to claim 11 further comprising opening an active EEG recording and occupying the entire display page with a single separate cell of the plurality of separate cells using a one-click function of the client device.

## Patentansprüche

1. System (100, 400) zum Überwachen mehrerer EEG-Maschinen und zum Erfassen von EEG-Aufzeichnung von jeder EEG-Maschine, wobei das System Folgendes aufweist:
eine Vielzahl von EEG-Maschinen (40), wobei jede der Vielzahl von EEG-Maschinen eine Vielzahl von Elektroden (30) zum Erzeugen einer Vielzahl von EEG-Signalen aufweist;
ein Netzwerk (450);
einen Client-Standort, der eine Verarbeitungsmaschine, auch Processing Engine genannt, eine Datenbank (420) und einen Anzeigemonitor aufweist, wobei die Verarbeitungsmaschine konfiguriert ist, um jedes der Vielzahl von EEG-Signalen von jeder der EEG-Maschinen zu verarbeiten, um eine Vielzahl von verarbeiteten EEG-Aufzeichnungen zu erzeugen, wobei die Verarbeitungsmaschine konfiguriert ist, um jede der EEG-Aufzeichnungen in einer separaten Zelle in einer Anzeigeseite (200) zur Anzeige auf dem Anzeigemonitor anzuzeigen;
**dadurch gekennzeichnet, dass** sich jede der Vielzahl von EEG-Maschinen an einem separaten Standort befindet und die Verarbeitungsmaschine konfiguriert ist, um jeden Kanal während der Erfassung der EEG-Signale auf Elektrodenartefakte zu überwachen und die Elektrodenartefakte zu entfernen, um ein höheres Maß an Anfall-Erkennungsgenauigkeit als ohne Entfernung der Elektrodenartefakte zu gewährleisten;
wobei die Verarbeitungsmaschine konfiguriert ist, um Artefakte in den EEG-Signalen durch Analysieren einer Vielzahl von Merkmalen zu detektieren;
wobei eine Artefaktintensität (110a) in jeder der Vielzahl von separaten Zellen dargestellt ist, wobei die Artefaktintensität als eine Reihe von horizontalen Linien dargestellt ist, die eine horizontale Linie für ein Muskelartefakt, eine horizontale Linie für ein Kauartefakt, eine horizontale Linie für ein vertikales Augenbewegungsartefakt und eine horizontale Linie für ein seitliches Augenbewegungsartefakt aufweist;
wobei die Verarbeitungsmaschine konfiguriert ist, um eine Vielzahl von Trends für jede der EEG-Aufzeichnungen in jeder der separaten Zellen auf der Anzeigeseite anzuzeigen, wobei die Vielzahl von Trends einen Anfall-Wahrscheinlichkeitstrend (120a) aufweist, der eine berechnete Wahrscheinlichkeit von Anfall-Aktivität über eine vorbestimmte Zeit anzeigt, ein Rhythmik-Spektrogramm, das die Größe an Rhythmik misst, die bei jeder Frequenz in einer EEG-Aufzeichnung vorhanden ist, einen Trend der linken Hemisphäre (130a), einen Trend der rechten Hemisphäre (140a), einen Fast-Fourier-Transformation-, FFT, Spektrogramm Trend der linken Hemisphäre (150a), einen FFT-Spektrogramm Trend der rechten Hemisphäre (160a), einen asymmetrie, relativ Spektrogramm-Trend und einen ambulant-EEG, aEEG, Trend (190a).

2. System nach Anspruch 1, wobei die Verarbeitungsmaschine konfiguriert ist, um einer neu aktiven EEG-Maschine eine separate Zelle zuzuordnen.

3. System nach Anspruch 1, wobei die Verarbeitungsmaschine konfiguriert ist, um das Umschalten zwischen mehreren Modi zu ermöglichen.

4. System nach Anspruch 1, wobei die Verarbeitungsmaschine konfiguriert ist, um jede der einzelnen Zellen in Echtzeit zu aktualisieren.

5. System nach Anspruch 1, wobei die Verarbeitungsmaschine konfiguriert ist, um mit einem Klick das Öffnen einer aktiven EEG-Aufzeichnung und das Belegen der gesamten Anzeigeseite mit der einzelnen separaten Zelle zu ermöglichen.

6. System nach Anspruch 1, wobei die Verarbeitungsmaschine konfiguriert ist, um eine Benutzerschnittstelle zuzuweisen.

7. System nach Anspruch 1, wobei die Verarbeitungsmaschine konfiguriert ist, um einen Parameter einer Vielzahl von Parametern einer Zelle zu ändern, ohne die Vielzahl von Parametern einer der anderen Zellen auf der Anzeigeseite zu beeinflussen.

8. System nach Anspruch 1, wobei die Verarbeitungsmaschine konfiguriert ist, um sich durch verschiedene offene EEG-Aufzeichnungen auf der Anzeigeseite zu bewegen, sie zu einer Teil-EEG-Aufzeichnung zu bewegen und mit einem Klick zu erlauben eine aktive EEG-Aufzeichnung zu öffnen und die gesamte Anzeigeseite mit der einzelnen separaten Zelle zu belegen.

9. System nach Anspruch 1, wobei die Verarbeitungsmaschine konfiguriert ist, um die Überprüfung einer gesamten EEG-Aufzeichnung auf der Anzeigeseite zu ermöglichen.

10. System nach Anspruch 1, wobei jede der EEG-Maschinen der Vielzahl von EEG-Maschinen ferner einen mit der Vielzahl von Elektroden verbundenen Prozessor zum Erzeugen einer EEG-Aufzeichnung aus der Vielzahl von EEG-Signalen und eine mit dem Prozessor verbundene Anzeige zum Anzeigen einer EEG-Aufzeichnung in der Nähe des Patienten aufweist.

11. Verfahren (500) zum Überwachen mehrerer EEG-Maschinen und zum Erfassen von EEG-Aufzeichnungen für jede der EEG-Maschinen, wobei das Verfahren Folgendes aufweist:
Überwachen (501) einer Vielzahl von EEG-Maschinen von einer zentralen Station aus, wobei jede der Vielzahl von EEG-Maschinen eine Vielzahl von Elektroden, einen Verstärker und einen Prozessor aufweist, wobei die zentrale Station mit jeder der Vielzahl von EEG-Maschinen über ein Netzwerk in Verbindung steht;
Erfassen (502) von EEG-Signalen von jeder aktiven EEG-Maschine der Vielzahl von EEG-Maschinen;
Übertragen der EEG-Signale über ein Netzwerk an eine Verarbeitungsmaschine an einer Client-Vorrichtung;
Verarbeiten (503) der EEG-Signale an der Verarbeitungsmaschine, um verarbeitete EEG-Aufzeichnungen für jede der aktiven EEG-Maschinen der Vielzahl von EEG-Maschinen und eine Vielzahl von Trends für jede der verarbeiteten EEG-Aufzeichnungen zu erzeugen;
Anzeigen (504) jeder der verarbeiteten EEG-Aufzeichnungen für jede der aktiven EEG-Maschinen der Vielzahl von EEG-Maschinen und wenigstens eines Trends der Vielzahl von Trends in einer separaten Zelle auf einer Anzeigeseite an einer Client-Vorrichtung;
**dadurch gekennzeichnet, dass** sich jede der Vielzahl von EEG-Maschinen an einem separaten Standort befindet und **gekennzeichnet durch** Überwachen durch die Verarbeitungsmaschine jedes Kanals auf Elektrodenartefakte während der Erfassung der EEG-Signale und Entfernen der Elektrodenartefakte, um ein höheres Maß an Anfall-Erkennungsgenauigkeit als ohne Entfernen der Elektrodenartefakte zu gewährleisten;
Detektieren von Artefakten in den EEG-Signalen durch die Verarbeitungsmaschine durch Analysieren einer Vielzahl von Merkmalen;
Darstellen einer Artefaktintensität in jeder der Vielzahl von getrennten Zellen, wobei die Artefaktintensität als eine Reihe von horizontalen Linien dargestellt ist, die eine horizontale Linie für ein Muskelartefakt, eine horizontale Linie für ein Kauartefakt, eine horizontale Linie für ein vertikales Augenbewegungsartefakt und eine horizontale Linie für ein seitliches Augenbewegungsartefakt aufweist;
wobei die Vielzahl von Trends Folgendes aufweist einen Anfall-Wahrscheinlichkeitstrend, der eine berechnete Wahrscheinlichkeit der Anfall-Aktivität über eine vorbestimmte Zeit anzeigt, ein Rhythmik-Spektrogramm, das die Größe der Rhythmik misst, welche bei jeder Frequenz in einer EEG-Aufzeichnung vorhanden ist, einen Trend der linken Hemisphäre, einen Trend der rechten Hemisphäre, einen Fast-Fourier-Transformation-, FFT, Spektrogramm Trend der linken Hemisphäre, einen FFT-Spektrogramm Trend der rechten Hemisphäre, einen asymmetrie, relativ Spektrogramm-Trend und einen ambulant-EEG, aEEG, Trend.

12. Verfahren nach Anspruch 11, ferner aufweisend das Zuweisen einer separaten Zelle aus der Vielzahl der separaten Zellen zu einer neu aktiven EEG-Maschine.

13. Verfahren nach Anspruch 11, ferner aufweisend das Umschalten zwischen mehreren Modi in jeder separaten Zelle der Vielzahl von separaten Zellen.

14. Verfahren nach Anspruch 11, ferner aufweisend das Aktualisieren jeder einzelnen Zelle der Vielzahl von separaten Zellen in Echtzeit.

15. Verfahren nach Anspruch 11, ferner aufweisend das Öffnen einer aktiven EEG-Aufzeichnung und Belegen der gesamten Anzeigeseite mit einer einzelnen separaten Zelle aus der Vielzahl der separaten Zellen unter Verwendung einer Ein-Klick-Funktion der Client-Vorrichtung.

## Revendications

1. Système (100, 400) pour surveiller plusieurs machines EEG et acquérir un enregistrement EEG provenant de chaque machine EEG, le système comprenant :
une pluralité de machines EEG (40), chaque machine de la pluralité de machines EEG comprenant une pluralité d'électrodes (30) pour générer une pluralité de signaux EEG ;
un réseau (450) ;
un site client comprenant un moteur de traitement, une base de données (420) et un moniteur d'affichage, le moteur de traitement étant configuré pour traiter chaque signal de la pluralité de signaux EEG provenant de chacune des machines EEG pour générer une pluralité d'enregistrements EEG traités, dans lequel le moteur de traitement est configuré pour afficher chacun des enregistrements EEG dans une cellule distincte d'une page d'affichage (200) à afficher sur le moniteur d'affichage ;
**caractérisé en ce que** chaque machine de la pluralité de machines EEG est dans une installation distincte et le moteur de traitement est configuré pour surveiller chaque canal vis-à-vis de parasites d'électrode pendant une acquisition de signaux EEG et supprime les parasites d'électrode pour fournir un niveau de fiabilité de détection de crise supérieur à un état sans suppression des parasites d'électrode ;
dans lequel le moteur de traitement est configuré pour détecter des parasites dans les signaux EEG en analysant une pluralité de caractéristiques ;
dans lequel une intensité parasite (110a) est illustrée dans chaque cellule de la pluralité de cellules distinctes, l'intensité parasite apparaissant comme une série de lignes horizontales comprenant une ligne horizontale pour un parasite musculaire, une ligne horizontale pour un parasite de mastication, une ligne horizontale pour un parasite de mouvement vertical d'un œil, et une ligne horizontale pour un parasite de mouvement latéral d'un œil ;
dans lequel le moteur de traitement est configuré pour afficher une pluralité de tendances pour chacun des enregistrements EEG dans chacune des cellules distinctes dans la page d'affichage, dans lequel la pluralité de tendances comprend une tendance de probabilité de crise (120a) montrant une probabilité calculée d'activité de crise pendant une période prédéterminée, un spectrogramme de périodicité mesurant la valeur de périodicité qui est présente pour chaque fréquence dans un enregistrement EEG, une tendance d'hémisphère gauche (130a), une tendance d'hémisphère droit (140a), une tendance d'hémisphère gauche (150a) de spectrogramme de transformée de Fourier rapide, FFT, une tendance d'hémisphère droit (160a) de spectrogramme de FFT, une tendance de spectrogramme relative d'asymétrie, et une tendance d'EEG ambulatoire, aEEG, (190a).

2. Système selon la revendication 1, dans lequel le moteur de traitement est configuré pour affecter une cellule distincte à une machine EEG nouvellement active.

3. Système selon la revendication 1, dans lequel le moteur de traitement est configuré pour autoriser la commutation entre les plusieurs modes.

4. Système selon la revendication 1, dans lequel le moteur de traitement est configuré pour mettre à jour chacune des cellules distinctes en temps réel.

5. Système selon la revendication 1, dans lequel le moteur de traitement est configuré pour permettre en un clic d'ouvrir un enregistrement EEG actif et occuper la page d'affichage entière avec la seule cellule distincte.

6. Système selon la revendication 1, dans lequel le moteur de traitement est configuré pour attribuer une interface utilisateur.

7. Système selon la revendication 1, dans lequel le moteur de traitement est configuré pour modifier un paramètre parmi une pluralité de paramètres d'une cellule sans affecter la pluralité de paramètres de l'une quelconque des autres cellules sur la page d'affichage.

8. Système selon la revendication 1, dans lequel le moteur de traitement est configuré pour se déplacer parmi différents enregistrements EEG ouverts sur la page d'affichage, pour se déplacer vers un enregistrement EEG partiel, et pour permettre en un clic d'ouvrir un enregistrement EEG actif et d'occuper la la page d'affichage entière avec la seule cellule distincte.

9. Système selon la revendication 1, dans lequel le moteur de traitement est configuré pour permettre la révision d'un enregistrement EEG complet sur la page d'affichage.

10. Système selon la revendication 1, dans lequel chacune des machines EEG de la pluralité de machines EEG comprend en outre un processeur connecté à la pluralité d'électrodes pour générer un enregistrement EEG à partir de la pluralité de signaux EEG et un affichage connecté au processeur pour afficher un enregistrement EEG à proximité du patient.

11. Procédé (500) de surveillance de plusieurs machines EEG et d'acquisition d'enregistrements EEG pour chacune des machines EEG, le procédé comprenant les étapes suivantes :
la surveillance (501) d'une pluralité de machines EEG à partir d'une station centrale, chaque machine parmi la pluralité des machines EEG comprenant une pluralité d'électrodes, un amplificateur et un processeur, la station centrale étant en communication avec chaque machine parmi la pluralité de machines EEG sur un réseau ;
l'acquisition (502) de signaux EEG à partir de chaque machine EEG active parmi la pluralité de machines EEG ;
l'émission des signaux EEG sur un réseau à destination d'un moteur de traitement au niveau d'un dispositif client ;
le traitement (503) des signaux EEG au niveau du moteur de traitement pour générer des enregistrements EEG traités pour chacune des machines EEG actives de la pluralité de machines EEG et une pluralité de tendances pour chacun des enregistrements EEG traités ;
l'affichage (504) de chacun des enregistrements EEG traités pour chacune des machines EEG actives de la pluralité de machines EEG et d'au moins une tendance parmi la pluralité de tendances dans une cellule distincte sur une page d'affichage au niveau d'un dispositif client ;
**caractérisé en ce que** chaque machine parmi la pluralité de machines EEG est dans une installation distincte et **caractérisé par** la surveillance, par le moteur de traitement, de chaque canal vis-à-vis de parasites d'électrodes pendant une acquisition des signaux EEG et la suppression des parasites d'électrode pour fournir un niveau de fiabilité de détection de crise supérieur au cas sans suppression des parasites d'électrodes ;
la détection, par le moteur de traitement, de parasites dans les signaux d'EEG en analysant une pluralité de caractéristiques ;
l'illustration d'une intensité de parasites dans chaque cellule parmi la pluralité de cellules distinctes, l'intensité de parasites étant montrée sous forme d'une série de lignes horizontales comprenant une ligne horizontale pour un parasite musculaire, une ligne horizontale pour un parasite de mastication, une ligne horizontale pour un parasite de mouvement vertical d'un œil, et une ligne horizontale pour un parasite de mouvement latéral d'un œil ;
dans lequel la pluralité de tendances comprend une tendance de probabilité de crise montrant une probabilité calculée d'activité de crise pendant une période prédéterminée, un spectrogramme de périodicité mesurant la valeur de périodicité qui est présente pour chaque fréquence dans un enregistrement EEG, une tendance d'hémisphère gauche, une tendance d'hémisphère droit, une tendance d'hémisphère gauche de spectrogramme de transformée de Fourier rapide, FFT, une tendance d'hémisphère droit de spectrogramme de FFT, une tendance de spectrogramme relative d'asymétrie, et une tendance d'EEG ambulatoire, aEEG.

12. Procédé selon la revendication 11, comprenant en outre l'affectation d'une cellule distincte parmi la pluralité de cellules distinctes à une machine EEG nouvellement active.

13. Procédé selon la revendication 11, comprenant en outre la commutation entre les plusieurs modes dans chaque cellule distincte parmi la pluralité de cellules distinctes.

14. Procédé selon la revendication 11, comprenant en outre la mise à jour de chaque cellule distincte parmi la pluralité de cellules distinctes en temps réel.

15. Procédé selon la revendication 11, comprenant en outre l'ouverture d'un enregistrement EEG actif et l'occupation de la page d'affichage entière avec une seule cellule distincte parmi la pluralité de cellules distinctes en utilisant une fonction en un clic du dispositif client.
